# EUROPÄISCHE PATENTSCHRIFT

(11) **EP 0 573 874 B1**
(45) Veröffentlichungstag und Bekanntmachung des Hinweises auf die Patenterteilung: **11.09.1996**
(21) Anmeldenummer: 93108757.1
(22) Anmeldetag: 01.06.1993
(51) Int. Cl.: C07C 43/20, C07C 41/16

(54) **Verfahren zur Herstellung von beta-Naphthylbenzylether**
Process for the preparation of beta-naphthylbenzylether
Procédé pour la préparation du bêta-naphtylbenzyléther

(30) Priorität: 06.06.1992 DE 4218767
(43) Veröffentlichungstag der Anmeldung: 15.12.1993
(73) Patentinhaber: BASF Aktiengesellschaft, 67063 Ludwigshafen (DE)
(72) Erfinder: Dyllick-Brenzinger, Rainer, Dr., W-6940 Weinheim (DE); Baus, Ulf, Dr., W-6915 Dossenheim (DE)

(56) Entgegenhaltungen:
- GB-A- 1 579 320
- JOURNAL OF THE AMERICAN CHEMICAL SOCIETY., Bd.42, 1920, GASTON, PA US Seiten 2059 - 2072 M. GOMBERG ET AL. 'The preparation of Benzyl esters and other benzyl Derivatives from Benzyl Chloride.'
- EUGEN MÜLLER 'Methoden der organischen Chemie, Band VI/3 Sauerstoffverbindungen I, Teil 3' 1965 , GEORG THIEME VERLAG , STUTTGART
- PATENT ABSTRACTS OF JAPAN vol. 6, no. 102 (C-107) 11. Juni 1982 & JP-A-57 031 631 (CHISSO CORP) 20. Februar 1982
- JOURNAL OF THE AMERICAN CHEMICAL SOCIETY., Bd.85, Nr.8, 20. April 1963, GASTON, PA US Seiten 1148 - 1154 NATHAN KORNBLUM ET AL. 'Solvation as a factor in the Alkylation of Ambident Anions: The Importance of the Dielectric Factor'
- CHEMICAL ABSTRACTS, vol. 71, no. 21, 24. November 1969, Columbus, Ohio, US; abstract no. 101529a, Seite 311 ;Spalte 2 ;

## Beschreibung

Die vorliegende Erfindung betrifft ein neues Verfahren zur Herstellung von β-Naphthylbenzylether durch Umsetzung von β-Naphthol im wesentlichen in Abwesenheit von Wasser mit einem Alkali- und/oder einem Erdalkalicarbonat und Benzylchlorid in einem inerten mit Wasser mischbaren Lösungsmittel.

Aus J.Am.Chem.Soc. Vol. 42, 2059 bis 2072 (1920) ist ein Verfahren zur Herstellung von β-Naphthylbenzylether durch Umsetzung des β-Naphthol-Natriumsalzes in wäßriger Lösung mit Benzylchlorid in 5%-iger Ausbeute bekannt.

Aus J.Am.Chem.Soc. Vol. 85, 1148 bis 1154 (1963) ist ein Verfahren zur Herstellung von β-Naphthylbenzylether durch Umsetzung des β-Naphthol-Natriumsalzes mit dem stark tränenreizenden Benzylbromid in Dimethylformamid oder Dimethylsulfoxid in Ausbeuten von 97% bzw. 95% bekannt.

Aus Chem. Abstr. Vol. 71, 101 529a (1969) ist die Umsetzung von β-Naphthol mit Benzylchlorid in Gegenwart von Oxiranen, Glycidethern und katalytischen Mengen von Aminen bekannt, die mit 60%-iger Ausbeute zum β-Naphthylbenzylether führt.

Die GB-A-1 579 320 beschreibt ein Verfahren zur Herstellung von β-Naphthylbenzylether mit bis zu 90% Ausbeute durch Umsetzung des β-Naphthol-Natriumsalzes mit Benzylbromid unter Verwendung von Phasentransferkatalysatoren.

Die bislang bekannten Verfahren haben den Nachteil, daß sie als Reaktanten das stark tränenreizende Benzylbromid verwenden bzw. lösliche und daher aufwendiger abtrennbare Hilfsstoffe mitverwenden. Ferner ließen die Ausbeuten z.T. zu wünschen übrig.

Der vorliegenden Erfindung lag daher die Aufgabe zugrunde, den zuvor genannten Nachteilen abzuhelfen.

Demgemäß wurde ein neues und verbessertes Verfahren zur Herstellung von β-Naphthylbenzylether gefunden, welches dadurch gekennzeichnet ist, daß man β-Naphthol im Wesentlichen in Abwesenheit von Wasser mit einem Alkali- und/oder einem Erdalkalicarbonat und Benzylchlorid in einem inerten mit Wasser mischbaren Lösungsmittel bei Temperaturen von 0 bis 200°C umsetzt.

Das erfindungsgemäße Verfahren läßt sich wie folgt durchführen:

β-Naphthol kann im Wesentlichen in Abwesenheit von Wasser, d.h. mit 0 bis 1 Gew.-%, bevorzugt 0 bis 0,1 Gew.-%, besonders bevorzugt 0 bis 0,01 Gew.-%, mit einem Alkali- und/oder einem Erdalkalicarbonat in einem inerten mit Wasser mischbaren Lösungsmittel bei Temperaturen von 0 bis 200°C, bevorzugt 60 bis 150°C, besonders bevorzugt 80 bis 120°C versetzt und anschließend Benzylchlorid zugesetzt, z.B. zugetropft werden.

Die Umsetzung kann im Wesentlichen in Abwesenheit, d.h. mit 0 bis 1 Gew.-%, bevorzugt 0 bis 0,1 Gew.-%, besonders bevorzugt 0 bis 0,01 Gew.-% bzw. insbesondere bevorzugt in Abwesenheit von Oxiranen, Glycidethern, Aminen oder Phasentransferkatalysatoren bei Drücken von 0,001 bis 50 bar, bevorzugt 0,1 bis 5 bar, besonders bevorzugt bei Normaldruck (Atmosphärendruck) durchgeführt werden.

Das Molverhältnis von β-Naphthol zu Benzylchlorid beträgt in der Regel 0,5 : 1 bis 1,5 : 1, bevorzugt 0,7 : 1 bis 1,2 : 1, besonders bevorzugt 0,8 : 1 bis 1,1 : 1.

Das Molverhältnis von β-Naphthol zum Alkali- und Erdalkalicarbonat beträgt in der Regel 0,01 : 1 bis 1 : 1, bevorzugt 0,1 : 1 bis 0,9 : 1, besonders bevorzugt 0,5 : 1 bis 0,8 : 1.

Als Alkali- und Erdalkalicarbonate eignen sich Lithiumcarbonat, Natriumcarbonat, Kaliumcarbonat, Rubidiumcarbonat, Caesiumcarbonat, Beryliumcarbonat, Magnesiumcarbonat, Calciumcarbonat, Strontiumcarbonat und Bariumcarbonat, bevorzugt Lithiumcarbonat, Natriumcarbonat, Kaliumcarbonat, Magnesiumcarbonat und Calciumcarbonat, besonders bevorzugt Natriumcarbonat, Kaliumcarbonat, Magnesiumcarbonat und Calciumcarbonat.

Als inerte mit Wasser mischbare Lösungsmittel eignen sich Ketone wie Aceton und Methyl-ethylketon, bevorzugt Aceton, cyclische Ether wie Tetrahydrofuran und Dioxan, Tetrahydrofuran, Nitrile wie Acetonitril, Sulfoxide wie Dimethylsulfoxid und/oder Formamide wie Dimethylformamid, Methylethylformamid, Diethylformamid, Dimethylacetamid und Diethylacetamid, bevorzugt Dimethylformamid.

Nach beendeter Reaktion kann der gebildete Rückstand (Salz) aus der Mischung abfiltriert werden und dem warmen (ca. 30 bis 100°C) Filtrat Wasser bis zur Trübung (d.h. zur beginnenden Kristallisation) zugegeben werden. Anschließend kann man die Mischung abkühlen lassen z.B. auf Raumtemperatur von 18 bis 25°C oder durch Kühlung auf 0 bis 10°C. Das ausgefallene Produkt, der β-Naphthyl-benzylether, kann abfiltriert und getrocknet werden. Das so erhaltene Produkt kann dabei in Ausbeuten über 90 %, häufig in Ausbeuten über 95 % in reiner Form mit 0 bis 3 Gew-%, bevorzugt 0 bis 1 Gew.-%, besonders bevorzugt 0 bis 0,5 Gew-% Verunreinigungen erhalten werden.

β-Naphthyl-benzylether findet z.B. für die Herstellung von Thermopapieren (Mitsubishi Paper) für beispielsweise Telefaxgeräte, Recorder usw. Verwendung. Die Funktion dieser Verbindung ist das Beschleunigen der Farbreaktion, ohne gleichzeitig an der Reaktion teilzunehmen (Lösemittel oder Sensitizer").

### Beispiele

### Beispiel 1

28,2g (200 mmol) β-Naphthol und 30,4g (220 mmol) Kaliumcarbonat wurden in 80 ml Dimethylformamid vermischt und 27,8 g (220 mmol) Benzylchlorid zugetropft und 2 Stunden auf 120°C erhitzt. Der entstandene Rückstand wurde abfiltriert und das Filtrat bis zur beginnenden Trübung mit Wasser versetzt. Man erhielt durch Filtration der erkalteten Mischung und Trocknung des Feststoffes 44,6 g (95 %) β-Naphthylbenzylether; Smp.: 101°C.

### Beispiele 2 bis 6

Analog Beispiel 1 wurde die Reaktion mit weiteren Lösungsmitteln durchgeführt. Menge der Einsatzstoffe und Lösungsmittel, Reaktionstemperatur und Reaktivität sowie die Ausbeuten sind in der folgenden Tabelle zusammengestellt.

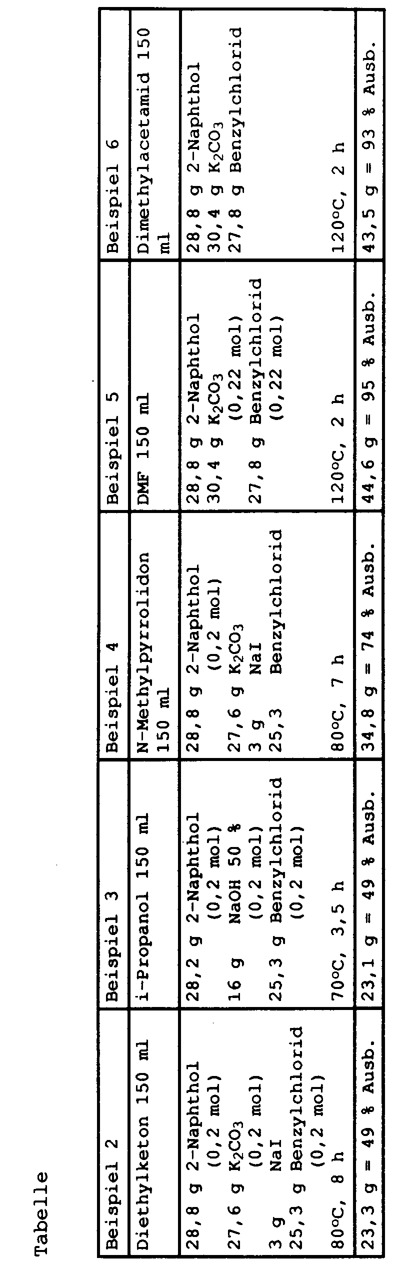

## Patentansprüche

1. Verfahren zur Herstellung von β-Naphthylbenzylether, dadurch gekennzeichnet, daß man β-Naphthol im Wesentlichen in Abwesenheit von Wasser,d.h. mit 0 bis 1 Gew.-%, mit einem Alkali- und/oder einem Erdalkalicarbonat und Benzylchlorid in einem inerten mit Wasser mischbaren Lösungsmittel bei Temperaturen von 0 bis 200°C umsetzt.

2. Verfahren zur Herstellung von β-Naphthylbenzylether nach Anspruch 1, dadurch gekennzeichnet, daß man die Umsetzung bei Temperaturen von 60 bis 150°C durchführt.

3. Verfahren zur Herstellung von β-Naphthylbenzylether nach Anspruch 1, dadurch gekennzeichnet, daß man als inertes mit Wasser mischbares Lösungsmittel Ketone, cyclische Ether, Nitrile Sulfoxide und/oder Formamide einsetzt.

4. Verfahren zur Herstellung von β-Naphthylbenzylether nach Anspruch 1, dadurch gekennzeichnet, daß man dem Reaktiongemisch nach der Umsetzung Wasser bis zur beginnenden Kristallisation zusetzt.

## Claims

1. A process for preparing β-naphthyl benzyl ether, which comprises reacting β-naphthol essentially in the absence of water, ie. 0-1% by weight of water, with an alkali metal carbonate and/or an alkaline earth metal carbonate and benzyl chloride in an inert water-miscible solvent at from 0 to 200°C.

2. A process for preparing β-naphthyl benzyl ether as claimed in claim 1, wherein the reaction is carried out at from 60 to 150°C.

3. A process for preparing β-naphthyl benzyl ether as claimed in claim 1, wherein the inert water-miscible solvent used is a ketone, a cyclic ether, a nitrile, a sulfoxide and/or a formamide.

4. A process for preparing β-naphthyl benzyl ether as claimed in claim 1, wherein, after the reaction has ended, the reaction mixture is admixed with water to the onset of crystallization.

## Revendications

1. Procédé de préparation d'éther β-naphtylbenzylique, caractérisé en ce que l'on fait réagir le β-naphtol sensiblement en l'absence d'eau, c'est-à-dire 0 à 1% en poids, avec un carbonate de métal alcalin ou de métal alcalino-terreux et le chlorure de benzyle dans un solvant inerte, miscible à l'eau, à des températures de 0 à 200°C.

2. Procédé de préparation d'éther β-naphtylbenzylique suivant la revendication 1, caractérisé en ce que l'on entreprend la réaction à des températures de 60 à 150°C.

3. Procédé de préparation d'éther β-naphtylbenzylique suivant la revendication 1, caractérisé en ce que l'on met en oeuvre, à titre de solvant inerte, miscible à l'eau, des cétones, des éthers cycliques, des nitriles, des sulfoxydes et/ou des formamides.

4. Procédé de préparation d'éther β-naphtylbenzylique suivant la revendication 1, caractérisé en ce qu'après la réaction, on ajoute de l'eau au mélange réactionnel jusqu'à cristallisation débutante.
